# EUROPÄISCHE PATENTSCHRIFT

(11) **EP 0 827 949 B1**
(45) Veröffentlichungstag und Bekanntmachung des Hinweises auf die Patenterteilung: **14.03.2001**
(21) Anmeldenummer: 97115374.7
(22) Anmeldetag: 04.09.1997
(51) Int. Cl.: C07C 209/60, C07C 211/23

(54) **Verfahren zur heterogen katalysierten Herstellung von n-alkyl-substituierten Aminoalkinen**
Process for the heterogeneous catalyzed preparation of n-alkyl substituted aminoalkines
Procédé pour la préparation par catalyse hétérogène des aminoalkines n-alkyl substitués

(30) Priorität: 05.09.1996 DE 19636078
(43) Veröffentlichungstag der Anmeldung: 11.03.1998
(73) Patentinhaber: BASF AKTIENGESELLSCHAFT, 67056 Ludwigshafen (DE)
(72) Erfinder: Preiss, Thomas, Dr., 67065 Ludwigshafen (DE); Henkelmann, Jochem, Dr., 68165 Mannheim (DE); Wulff-Döring, Joachim, Dr., 67227 Frankenthal (DE); Stutz, Susanne, Dr., 69469 Weinheim (DE)
(74) Vertreter: Kinzebach, Werner, Dr.

(56) Entgegenhaltungen:
- EP-A- 0 080 794
- EP-A- 0 478 977
- US-A- 3 496 232
- US-A- 3 650 985
- US-A- 4 127 734

## Beschreibung

Die vorliegende Erfindung betrifft ein heterogen katalysiertes Verfahren zur Herstellung von N-Alkyl-substituierten Aminoalkinen in Gegenwart eines ungeträgerten Katalysators.

N-Alkylaminoalkine sind wichtige industrielle Zwischenprodukte, die vielfach Verwendung finden. Sie dienen teilweise als Vorprodukte für Pharmazeutika, werden allerdings auch in der Galvanik oder als Korrosionsinhibitoren eingesetzt.

Die Herstellung von N-Alkyl-substituierten Aminoalkinen ist seit langem bekannt und wird industriell genutzt. Dabei werden im allgemeinen entsprechend substituierte Alkine, Carbonylverbindungen und Amine in einer homogen oder heterogen katalysierten Reaktion im Sinne einer Mannich-Kondensation umgesetzt.

Homogen katalysierte Verfahren dieses Typs sind weit verbreitet und vielfach beschrieben. So beschreibt z.B. die CH-A-669 192 die Herstellung von pharmakologisch wirksamen N-Arylalkyl-substituierten Aminoalkinen in einer homogen katalysierten Reaktion, wobei als Katalysatoren Kupfer- und Zinksalze, wie z.B. CuCl oder ZnCl₂, eingesetzt werden.

Die DE-A-26 37 425 beschreibt die Herstellung von Dialkylamino-2-alkin-4-olen durch Umsetzung von Formaldehyd, Dialkylamin und einem Alkinol in wässriger, saurer Lösung, bevorzugt bei einem pH von 5, unter Verwendung eines speziellen Katalysatorsystems, nämlich einer Kombination von im Reaktionsgemisch löslichen Bromiden, Iodiden oder Jod und löslichen Cu(II)-Verbindungen. Die heterogen katalysierte Durchführung der Reaktion im neutralen oder alkalischen pH-Bereich wird nicht als mögliche Variante diskutiert. Die DE-B-1 100 617 beschreibt ebenfalls die Herstellung von Dialkylamino-2-alkin-4-olen durch Umsetzung von Formaldehyd, Dialkylamin und einem Alkinol in wässriger, saurer Lösung, bevorzugt bei einem pH von 5 bis 6 und homogene Katalyse mit Kupfersulfat, -acetat, -nitrat oder -chlorid.

Diese Verfahren weisen die bekannten, mit der Abtrennung des homogenen Katalysators aus dem Reaktionsgemisch verbundenen Nachteile auf.

Außerdem sind diese Verfahren nicht anwendbar bei Verwendung leicht flüchtiger Reaktanden, wie insbesondere niedrig siedender Alkine.

Die Herstellung von Aminoalkinen unter Verwendung leicht flüchtiger Reaktanden ist dagegen experimentell deutlich aufwendiger.

Die US-A-3,496,232 beschreibt z.B. die Herstellung von Propargylaminen mit Hilfe der Mannich-Reaktion. Als Katalysatoren werden zwar gegebenenfalls geträgerte Salze von Metallen der ersten oder zweiten Nebengruppe, wie z.B. die Chloride, Acetate und Formiate von Kupfer allgemein beschrieben. Bevorzugt erfolgt die Durchführung der Reaktion jedoch durch homogene Katalyse mit CuCl₂. Dieses Verfahren besitzt den Nachteil, daß es technisch aufwendig mit verflüssigtem Acetylen bei hohen Drücken (25 bis 70 atm) durchgeführt werden muß und keine zufriedenstellenden Ausbeuten an Produkt liefert.

Die US-A-3,496,232 erwähnt zwar auch die Brauchbarkeit von Kupferacetylid-Katalysatoren. Da diese zur explosionsartigen Zersetzung neigen, nur schwer durch Filtration von der Reaktionslösung abzutrennen sind und zudem die Bildung von Cupren, einem Acetylenpolymerisationsprodukt, katalysieren, sind diese Katalysatoren nicht bevorzugt.

Zur leichteren Handhabung von Kupferacetyliden werden diese auf einen inerten Träger aufgetragen und mit einer Bismutverbindung versetzt, um die Cuprenbildung zu verringern. Der Einsatz solcher bekannter Katalysatoren zur Aminoalkylierung von unter den Reaktionsbedingungen gasförmigen Alkinen erfordert jedoch hohe Partialdrücke um eine annähernd akzeptable Raum-Zeit-Ausbeute zu erzielen. Beim Arbeiten mit Acetylen, einem thermisch instabilen, bereits bei Normaldruck leicht explodierenden Gas, sind erhebliche sicherheitstechnische Maßnahmen zur Auslegung der Reaktoren für die erforderlichen Druckbereiche notwendig, was diese Verfahren wirtschaftlich benachteiligt.

So beschreibt z.B. die EP-A-0 080 794 ein heterogen katalysiertes Verfahren zur Herstellung von N,N-disubstituierten Propinylaminen, wobei als bevorzugte Katalysatoren Kupferacetylide auf einem mit Bismutoxid dotierten Magnesiumsilikatträger eingesetzt werden. Die Reaktion erfolgt z.B. in einem Rührautoklaven mit einem aufgeschlämmten Katalysator oder in einem Festbett. Dieses Verfahren besitzt den Nachteil, daß der verwendete geträgerte Katalysator aufwendig herzustellen ist und wegen seines geringen Kupfergehaltes (etwa 5 bis 35 %) keine zufriedenstellende Aktivität besitzt. Die Umsetzung von Acetylen erfordert hierbei Partialdrücke von bis zu 20 oder mehr atm.

Aufgrund der geschilderten Nachteile ist man deshalb auf der Suche nach einem verbesserten Verfahren zur Herstellung von Aminoalkinen.

Die US-A-3,650,985 beschreibt die Herstellung trägerloser Kupferacetylid-Katalysatoren, der allgemeinen Formel (CuC₂)_{w} (CH₂O)ₓ (C₂H₂)_{y} (H₂O)_{z} mit 1 ≤ w, x, y < 100, vorzugsweise w = 4, x = 0,24 bis 4, y = 0,24 bis 4 und z = 0,67 bis 2,8. Diese Katalysatoren können zusätzlich eine Bismutverbindung enthalten und sind durch simultanes Einwirken von Formaldehyd und Acetylen auf eine partikuläre, wasserunlösliche Kupferverbindung, vorzugsweise basisches Kupfercarbonat, wie z.B. synthetisch hergestellter Malachit herstellbar. Sie werden als wäßrige Katalysatoraufschlämmung zur Ethinylierung acetylenisch ungesättigter Kohlenwasserstoffe verwendet. Ähnliche Malachit-Katalysatoren werden in der US-A-3,560,576 beschrieben.

Die US-A-4,127,734 beschreibt die Herstellung von Bismut-modifizierten, kugelförmigen Malachiten und ihre Umsetzung mit Acetylen und Formaldehyd zu trägerlosen Ethinylierungskatalysatoren.

Weder in der US-A-3,650,985 noch in der US-A-4,127,734 wird jedoch vorgeschlagen, diese speziellen Katalysatoren in anderen Reaktionen zu verwenden. Insbesondere fehlt jeglicher Hinweis auf eine mögliche Brauchbarkeit dieser Katalysatoren für die Herstellung von Aminoalkinen in nichtwässrigem Milieu.

Der vorliegenden Erfindung lag deshalb die Aufgabe zugrunde, ein Verfahren zur Herstellung von N-Alkyl-substituierten Aminoalkinen zur Verfügung zu stelle, das die aus dem Stand der Technik bekannten Nachteile nicht mehr aufweist. Insbesondere sollte das neue Verfahren ein Arbeiten bei möglichst niedrigen Drücken ermöglichen. Außerdem sollten mit dem Verfahren die gewünschten Aminoalkine in hohen Ausbeuten und mit hoher Selektivität bei einer niedrigen Katalysatoraufwandmenge herstellbar sein.

Überraschenderweise wurde nun gefunden, daß diese Aufgabe durch die Bereitstellung eines Verfahrens gelöst wird, bei dem ein Alkin mit einer Carbonylverbindung und einem Amin in einer heterogen katalysierten Reaktion mit einem trägerlosen Kupfer-Katalysator umgesetzt wird, der von Malachit abgeleitet ist.

Überraschenderweise wurde weiterhin gefunden, daß das erfindungsgemäße Verfahren, entgegen der Erwartung des Fachmanns, auch zur Herstellung von Aminoalkinen, die am Stickstoffatom und/oder am Alkin-Kohlenstoffatom Hydroxyalkyl-substituiert sind geeignet ist, obwohl dabei mit einer ganzen Reihe von Nebenreaktionen zu rechnen ist. Beim Einsatz von Mono- oder Di-Hydroxyalkyl-substituierten Aminen und von einer Carbonylverbindung ist mit Ringschlußreaktionen zu rechnen, wie z.B. der Oxazolidinbildung. Dies gilt insbesondere beim Einsatz von β-Aminoalkoholen als Aminkomponente, wenn unter alkalischen Reaktionsbedingungen in wasserfreien Lösungsmitteln oder in Gegenwart von wasserentziehenden Mitteln gearbeitet wird, und nicht, wie in der DE-A-26 37 425 beschrieben, in saurer, wäßriger Lösung. Außerdem ist mit der Acetalbildung aus Hydroxyalkyl-substituierten Reaktanden und der Carbonylverbindung zu rechnen. Diese Acetale besitzen unter alkalischen Reaktionsbedingungen erhöhte Stabilität. Des weiteren kann es auch zur Bildung offenkettiger Kondensationsprodukte aus einem Hydroxyalkyl-substituierten Alkin, der Aminkomponente und einem entsprechenden Aldehyd kommen. Überraschenderweise wurde dabei außerdem festgestellt, daß keine besonderen Vorkehrungen zu pH-Wert-Einstellung zu treffen sind; es kann nämlich in dem durch die Reaktanden vorgegebenen neutralen bis alkalischen pH-Wert-Bereich gearbeitet werden, ohne daß es zu den erwarteten unerwünschten Nebenreaktionen kommt.

Gegenstand der vorliegenden Erfindung ist ein Verfahren zur Herstellung von N-Alkyl-substituierten Aminoalkinen der allgemeinen Formel I 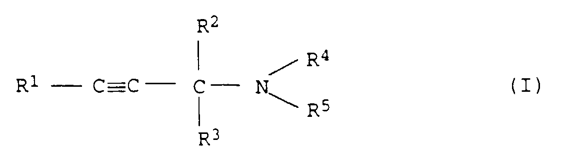 worin
- R¹: für ein Wasserstoffatom, Alkyl, Halogenalkyl, Cycloalkyl, Aryl, Alkoxy, Alkoxyalkyl oder Hydroxyalkyl, vorzugsweise für ein Wasserstoffatom, Alkyl oder Hydroxyalkyl steht;
- R² und R³: unabhängig voneinander für ein Wasserstoffatom, Alkyl, Halogenalkyl, Aryl oder Alkoxy stehen, wobei insbesondere wenigstens einer der Reste, vorzugsweise beide, für ein Wasserstoffatom stehen;
- R⁴ und R⁵: unabhängig voneinander für ein Wasserstoffatom, Alkyl, Halogenalkyl, Aryl, Alkoxy oder Hydroxyalkyl stehen, oder
R⁴ und R⁵ zusammen mit dem Stickstoffatom, an das sie gebunden sind, einen 5- oder 6-gliedrigen heterocyclischen Ring bilden,
wobei insbesondere wenigstens einer, vorzugsweise beide Reste für ein wasserstoffatom, Alkyl oder Hydroxyalkyl stehen;
wobei man ein Gemisch aus
einem Alkin der allgemeinen Formel II

R¹― C≡C― H (II)

worin
R¹ die oben angegebenen Bedeutungen besitzt,
einer Carbonylverbindung der allgemeinen Formel III 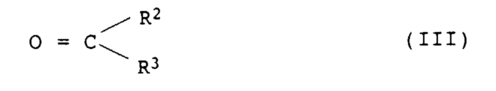 worin
R² und R³ die oben angegebenen Bedeutungen besitzen,
und einem Amin der allgemeinen Formel IV 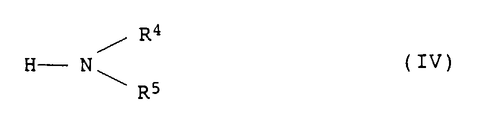 worin
R⁴ und R⁵ die oben angegebenen Bedeutungen besitzen,
in einer heterogen katalysierten Reaktion umsetzt, das dadurch gekennzeichnet ist, daß ein trägerloser Kupferacetylid-Katalysator verwendet wird, der von Malachit (CuCO₃·Cu(OH)₂) abgeleitet ist und wobei der Katalysator mindestens einen Kupferacetylid-Komplex der allgemeinen Formel V

(CuC₂)_{w} (CH₂O)ₓ (C₂H₂)_{y} (H₂O)_{z} (V)

mit 1 ≤ w, x, y, z < 100,
und eine Bismutverbindung enthält. Vorzugsweise setzt man den Katalysator in aktivierter Form, wie z.B. als Kupferacetylid-Katalysator ein. Es ist weiterhin denkbar, daß anstelle von Acetylen andere Alkine oder Alkinole zur Aktivierung verwendet werden.

Im Rahmen der vorliegenden Erfindung steht Halogen für Fluor, Chlor, Brom und Jod und insbesondere für Chlor und Brom.

Der Ausdruck "Alkyl" umfasst geradkettige und verzweigte Alkylgruppen. Vorzugsweise handelt es sich dabei um geradkettige oder verzweigte C₁-C₁₂-Alkyl- und insbesondere C₁-C₆-Alkylgruppen. Beispiele für Alkylgruppen sind insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 1-Ethylpentyl, 2-Ethylpentyl, 1-Propylbutyl, Octyl, Decyl und Dodecyl.

Halogenalkyl steht für eine wie oben definierte Alkylgruppe, die mit einem oder mehreren Halogenatomen, insbesondere Chlor und Brom, teilweise oder vollständig, vorzugsweise mit ein bis drei Halogenatomen, halogeniert ist.

Die obigen Ausführungen zur Alkylgruppe und Halogenalkylgruppe gelten in entsprechender Weise für die Alkylgruppe in Alkoxy-, Alkoxyalkyl- und Hydroxyalkyl-Resten.

Cycloalkyl steht vorzugsweise für C₃-C₈-Cycloalkyl, wie Cyclopropyl, Cyclobutyl, Cyclopentyl, Cyclohexyl, Cycloheptyl oder Cyclooctyl, oder Cyclopentylmethyl, Cyclopentylethyl und Cyclohexylmethyl und Cyclohexylethyl.

Aryl steht vorzugsweise für Phenyl oder Naphthyl.

Die Reste R⁴ und R⁵ können zusammen mit dem Stickstoffatom, an das sie gebunden sind, einen heterocyclischen Ring bilden. Beispiele hierfür sind Succinimido-, Phthalimido-Gruppen oder ein ungesättigter oder gesättigter 5- oder 6-gliedriger heterocyclischer Ring, der gegebenenfalls ein weiteres Heteroatom, ausgewählt unter S und N, vorzugsweise N, enthält. Als Beispiele können hierfür genannt werden: Piperidinyl-, Piperazinyl- und Tetrahydropyrimidinyl-Gruppen.

Man verwendet im erfindungsgemäßen Verfahren Kupferacetylid-Katalysatoren, die zusätzlich eine Bismutverbindung, wie z.B. (BiO)₂CO₃, Bi(NO₃)₃ oder BiO(NO₃) enthalten. Besonders bevorzugt sind Katalysatoren mit etwa 40 bis 70 Gew.-% Cu und etwa 0,1 bis 10 Gew.-% Bi.

Solche Katalysatoren sind aus dem Stand der Technik bekannt und werden z.B. in der US-A-3,650,985, in der US-A-3,560,576 und in der US-A-4,127,734 beschrieben. Auf die Offenbarung dieser Druckschriften wird hiermit ausdrücklich Bezug genommen.

In der vorliegenden Erfindung wird ein Katalysator verwendet, der mindestens einen Kupferacetylidkomplex der allgemeinen Formel V

(CuC₂)_{w} (CH₂O)ₓ (C₂H₂)_{y} (H₂O)_{z} (V)

mit 1 ≤ w, x, y, z < 100, und eine Bismutverbindung umfasst.

Vorzugsweise besitzen dabei die Indices folggende Werte:
- w: 2 bis 6, insbesondere 4,
- x: 0,24 bis 4,00,
- y: 0,24 bis 2,40,
- z: 0,67 bis 2,8.

Verfahren zur Herstellung dieser Bi-dotierten Kupferacetylidkomplexe sind ebenfalls in der US-A-3,650,985 und in der US-A-4,127,734 beschrieben. Allgemein werden die erfindungsgemäß als trägerlose heterogene Katalysatoren verwendeten Kupferacetylidkomplexe durch gleichzeitige Reaktion einer Kupferverbindung, ausgewählt unter Kupferoxiden, Kupfersilikaten, Kupferphosphaten, Kupferhydroxiden und basischen Kupfercarbonaten, wie z.B. natürlichen und bevorzugt synthetischen Malachiten, in Gegenwart einer Bismutverbindung, ausgewählt unter Bismutoxidcarbonat und Bismutnitrat, und gegebenenfalls in Gegenwart eines Alkalimetallcarbonats oder -hydrogencarbonats, mit Formaldehyd und Acetylen erhalten.

Gemäß einer bevorzugten Ausführungsform des erfindungsgemäßen Verfahrens werden Verbindungen hergestellt, worin wenigstens einer der Reste R¹, R⁴ oder R⁵ für Hydroxyalkyl steht. Überraschenderweise gelingt die Herstellung solcher Hydroxyalkyl-substituierter Aminoalkine ohne besondere Vorkehrungen zur Einstellung des pH-Wertes, nämlich in dem durch die Reaktanten vorgegebenen neutralen bis alkalischen pH-Bereich, ohne daß es zu den erwartenden unerwünschten Nebenreaktionen kommt.

Gemäß einer weiteren bevorzugten Ausführungsform des erfindungsgemäßen Verfahrens werden Verbindungen hergestellt, worin die Reste R² und R³ in der Formel III unabhängig voneinander für ein Wasserstoffatom oder Alkyl stehen. Insbesondere stehen die Reste R² und R³ beide für Wasserstoff.

Weiterhin werden bevorzugt Alkine der Formel II umgesetzt, worin R¹ für ein Wasserstoffatom, Alkyl oder Hydroxyalkyl steht.

Besonders eignet sich das erfindungsgemäße Verfahren zur Umsetzung von bei der jeweiligen Reaktionstemperatur gasförmigen Alkinen der Formel II, wie z.B. Acetylen, Propin, 1-Butin, etc. Vorzugsweise wird Acetylen verwendet. Vorteilhafterweise gelingt die Umsetzung dieser gasförmigen Alkine bei einem geringeren Druck als bei den aus dem Stand der Technik bekannten Verfahren, nämlich bei einem Druck von bis zu 3 bar, bevorzugt bis zu 2 bar und insbesondere bevorzugt bei Umgebungsdruck.

Wenn als Alkin der Formel II Acetylen verwendet wird, so wird dieses für die Umsetzung vorzugsweise weder komprimiert noch verflüssigt. Werden als Amine der Formel IV niedrigsiedende Amine verwendet, so kann die Reaktion gewünschtenfalls unter Eigendruck bei Einhaltung der vorbeschriebenen Druckbedingungen durchgeführt werden.

Ist die Verbindung der Formel II ein unter den Reaktionsbedingungen gasförmiges Alkin, speziell Acetylen, so wird die Carbonylverbindung der Formel III und das Amin der Formel IV gemeinsam mit dem trägerlosen Heterogenkatalysator und gegebenenfalls mit einem Lösungsmittel in einem mit einer Mischvorrichtung versehenen Reaktor vorgelegt. Geeignete Reaktoren sind dem Fachmann bekannt. Zu ihnen zählen die in Ullmanns Enzyklopädie der technischen Chemie, 3. Aufl., Band 1, S. 117 ff und S. 769 ff (1951) beschriebenen Reaktionsbehälter für Reaktionen unter Druck. Die Zugabe des Alkins erfolgt vorzugsweise unterhalb des Flüssigkeitsspiegels der vorgelegten Reaktionsmischung, z.B. mit einem Tauchrohr oder mit einer Rohrschlange, die Öffnungsbohrungen in der, oder gegen die Strömungsrichtung der Reaktionsmischung aufweist. Die Zugabegeschwindigkeit wird durch die vorgenannten einzuhaltenden Druckbereiche limitiert.

Die Reaktion kann lösungsmittelfrei oder in Gegenwart eines gegenüber den Reaktanden inerten organischen Lösungsmittels durchgeführt werden. Geeignete Lösungsmittel sind z.B. gesättigte cyclische Ether, wie z.B. Tetrahydrofuran und Dioxan.

Die Reaktionstemperatur kann im Bereich zwischen Umgebungstemperatur und der Siedetemperatur der Reaktionsmischung gewählt werden. Vorzugsweise erfolgt die Umsetzung bei einer Temperatur von 20 bis 200°C, bevorzugt 30 bis 180°C, insbesondere bevorzugt 40 bis 160°C.

Der pH-Wert der Reaktion wird durch die Reaktanden vorgegeben und liegt im neutralen oder alkalischen pH-Bereich.

Das erfindungsgemäße Verfahren zur Herstellung von N-Alkyl-substituierten Aminoalkinen unter Verwendung der genannten trägerlosen Heterogenkatalysatoren ermöglicht Umsetzungen im Sinne einer Mannich-Kondensation mit hohen Selektivitäten und hohen Ausbeuten.

Die Erfindung wird anhand der folgenden, nicht einschränkenden Beispiele erläutert.

### Beispiele

### Beispiel 1:

In einem 500 ml Dreihalskolben wurden 73 g (1 mol) Diethylamin mit 30 g (1 mol) Paraformaldehyd und 100 ml 1,4-Dioxan vorgelegt. Zu dieser Lösung wurden dann 4,5 g eines unter Normaldruck mit Acetylen aktivierten Kupferkatalysator (54 % Kupfer und 3 % Bismut) gegeben. Diese Lösung wurde dann auf 50°C erwärmt und 24 Stunden bei dieser Temperatur mit 6 l/Stunde Acetylen begast. Nach beendeter Reaktion wurde N,N-Diethylaminopropin in 90 % Ausbeutet (bezogen auf das Amin / Selektivität > 95 %) erhalten.

### Beispiel 2

In einem 500 ml Dreihalskolben wurden 129 g (1 mol) Di-n-Butylamin mit 30 g (1 mol) Paraformaldehyd vorgelegt. Zu dieser Lösung wurden dann 4,5 g eines unter Normaldruck mit Acetylen aktivierten Kupferkatalysator (54 % Kupfer und 3 % Bismut) gegeben. Diese Lösung wurde dann auf 80°C erwärmt und 16 Stunden bei dieser Temperatur mit 6 l/Stunde Acetylen begast. Nach beendeter Reaktion wurde N,N-Di-n-butylaminopropin in 92 % Ausbeute (bezogen auf das Amin / Selektivität > 95 %) erhalten.

## Patentansprüche

1. Verfahren zur Herstellung von N-Alkyl-substituierten Aminoalkinen der allgemeinen Formel I 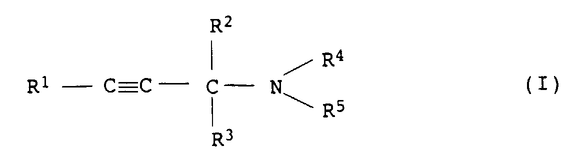 worin
R¹ für ein Wasserstoffatom, Alkyl, Halogenalkyl, Cycloalkyl, Aryl, Alkoxy, Alkoxyalkyl oder Hydroxyalkyl steht;
R² und R³ unabhängig voneinander für ein Wasserstoffatom, Alkyl, Halogenalkyl, Aryl oder Alkoxy stehen;
R⁴ und R⁵ unabhängig voneinander für ein Wasserstoffatom, Alkyl, Halogenalkyl, Aryl, Alkoxy oder Hydroxyalkyl stehen, oder
R⁴ und R⁵ zusammen mit dem Stickstoffatom, an das sie gebunden sind, einen 5- oder 6-gliedrigen heterocyclischen Ring bilden;
wobei man ein Gemisch aus
einem Alkin der allgemeinen Formel II
R¹― C≡C― H (II)
worin
R¹ die oben angegebenen Bedeutungen besitzt,
einer Carbonylverbindung der allgemeinen Formel III 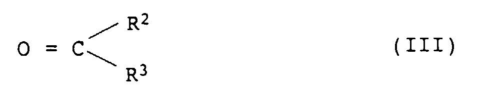 worin
R² und R³ die oben angegebenen Bedeutungen besitzen,
und einem Amin der allgemeinen Formel IV 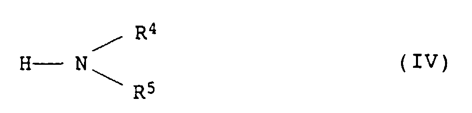 worin R⁴ und R⁵ die oben angegebenen Bedeutungen besitzen, in einer heterogen katalysierten Reaktion umsetzt, dadurch gekennzeichnet, daß ein trägerloser Kupferacetylid-Katalysator verwendet wird, der von Malachit abgeleitet ist und wobei der Katalysator mindestens einen Kupferacetylid-Komplex der allgemeinen Formel V
(CuC₂)_{w} (CH₂O)ₓ (C₂H₂)_{y} (H₂O)_{z} (V)
mit 1 ≤ w, x, y, z < 100,
und eine Bismutverbindung enthält.

2. Verfahren nach Anspruch 1, dadurch gekennzeichnet, daß der Katalysator 40 bis 70 Gew.-% Kupfer und 0,1 bis 10 Gew.-% Bismut enthält.

3. Verfahren nach einem der vorhergehenden Ansprüche, dadurch gekennzeichnet, daß man einen Komplex der Formel V verwendet, worin
w für einen Wert von 2 bis 6 steht,
x für einen Wert von 0,24 bis 4,00 steht,
y für einen Wert von 0,24 bis 2,40 steht, und
z für einen Wert von 0,67 bis 2,8 steht.

4. Verfahren nach einem der vorhergehenden Ansprüche, dadurch gekennzeichnet, daß die Reaktion bei einem Druck von bis zu 3 bar, bevorzugt bis zu 2 bar, insbesondere bevorzugt bei Umgebungsdruck durchgeführt wird.

5. Verfahren nach einem der vorhergehenden Ansprüche, dadurch gekennzeichnet, daß die Umsetzung bei einer Temperatur von 20 bis 200°C, bevorzugt 30 bis 180°C, insbesondere 40 bis 160°C durchgeführt wird.

6. Verfahren nach einem der vorhergehenden Ansprüche, dadurch gekennzeichnet, daß die Umsetzung im neutralen oder alkalischen pH-Bereich erfolgt.

7. Verfahren nach einem der vorhergehenden Ansprüche, dadurch gekennzeichnet, daß die Umsetzung in Substanz erfolgt.

8. Verfahren nach einem der vorhergehenden Ansprüche, dadurch gekennzeichnet, daß die Umsetzung in nichtwäßrigem Milieu erfolgt.

9. Verfahren nach einem der vorhergehenden Ansprüche, dadurch gekennzeichnet, daß man zuerst die Carbonylverbindung der Formel III, das Amin der Formel IV und den Katalysator, gegebenenfalls in einem Lösungsmittel, vorlegt und anschließend das Alkin der Formel II, vorzugsweise unterhalb des Flüssigkeitsspiegels der Reaktionsmischung, zugibt.

10. Verfahren nach einem der vorhergehenden Ansprüche, dadurch gekennzeichnet, daß man ein Alkin der Formel II umsetzt, worin R¹ für ein Wasserstoffatom, Alkyl oder Hydroxyalkyl steht.

11. Verfahren nach Anspruch 10, dadurch gekennzeichnet, daß als Verbindung der Formel II ein bei der Reaktionstemperatur gasförmiges Alkin verwendet wird.

12. Verfahren nach Anspruch 11, dadurch gekennzeichnet, daß als Verbindung der Formel II Acetylen verwendet wird, das für die Umsetzung nicht komprimiert oder verflüssigt wird.

13. Verfahren nach einem der Ansprüche 1 bis 9, dadurch gekennzeichnet, daß man ein Alkin der Formel II und ein Amin der Formel IV umsetzt, worin wenigstens einer der Reste R¹, R⁴ oder R⁵ für Hydroxyalkyl steht.

14. Verfahren nach einem der vorhergehenden Ansprüche, dadurch gekennzeichnet, daß man eine Carbonylverbindung der Formel III umsetzt, worin R² und R³ unabhängig voneinander für ein Wasserstoffatom oder Alkyl stehen, bevorzugt R² und R³ beide für ein Wasserstoffatom stehen.

## Claims

1. A process for preparing N-alkyl-substituted aminoalkynes of the general formula I 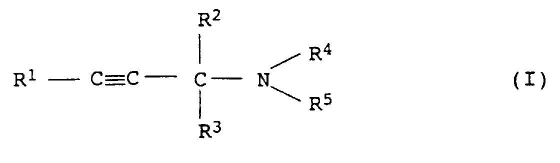 where
R¹ is hydrogen, alkyl, haloalkyl, cycloalkyl, aryl, alkoxy, alkoxyalkyl or hydroxyalkyl;
R² and R³ are, independently of one another, hydrogen, alkyl, haloalkyl, aryl or alkoxy;
R⁴ and R⁵ are, independently of one another, hydrogen, alkyl, haloalkyl, aryl, alkoxy or hydroxyalkyl, or
R⁴ and R⁵ form, together with the nitrogen atom to which they are bonded, a 5- or 6-membered heterocyclic ring;
wherein a mixture of
an alkyne of the general formula II
R¹― C≡C― H (II)
where
R¹ has the abovementioned meanings,
a carbonyl compound of the general formula III 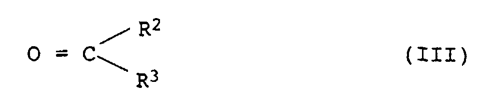 where
R² and R³ have the abovementioned meanings,
and an amine of the general formula IV 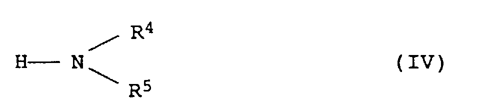 where R⁴ and R⁵ have the abovementioned meanings, is reacted with heterogeneous catalysis, wherein an unsupported copper acetylide catalyst which is derived from malachite is used and wherein the catalyst contains at least one copper acetylide complex of the general formula V
(CuC₂)_{w} (CH₂O)ₓ (C₂H₂)_{y} (H₂O)_{z} (V)
with 1 < w, x, y, z < 100,
and a bismuth compound.

2. A process as claimed in claim 1, wherein the catalyst contains 40 - 70 % by weight of copper and 0.1 - 10 % by weight of bismuth.

3. A process as claimed in either of the preceding claims, wherein a complex of the formula V where
w has a value from 2 to 6,
x has a value from 0.24 to 4.00,
y has a value from 0.24 to 2.40, and
z has a value from 0.67 to 2.8, is used.

4. A process as claimed in any of the preceding claims, wherein the reaction is carried out under a pressure of up to 3 bar, preferably up to 2 bar, particularly preferably under ambient pressure.

5. A process as claimed in any of the preceding claims, wherein the reaction is carried out at from 20 to 200°C, preferably 30 to 180°C, in particular 40 to 160°C.

6. A process as claimed in any of the preceding claims, wherein the reaction takes place in the neutral or alkaline pH range.

7. A process as claimed in any of the preceding claims, wherein the reaction takes place without diluent.

8. A process as claimed in any of the preceding claims, wherein the reaction takes place in a nonaqueous medium.

9. A process as claimed in any of the preceding claims, wherein first the carbonyl compound of the formula III, the amine of the formula IV and the catalyst are introduced, with or without a solvent, and subsequently the alkyne of the formula II is added, preferably beneath the level of the liquid reaction mixture.

10. A process as claimed in any of the preceding claims, wherein an alkyne of the formula II where R¹ is hydrogen, alkyl or hydroxyalkyl is reacted.

11. A process as claimed in claim 10, wherein an alkyne which is gaseous at the reaction temperature is used as compound of the formula II.

12. A process as claimed in claim 11, wherein acetylene is used as compound of the formula II and is not compressed or liquefied for the reaction.

13. A process as claimed in any of claims 1 to 9, wherein an alkyne of the formula II and an amine of the formula IV, where at least one of the radicals R¹, R⁴ or R⁵ is hydroxyalkyl, are reacted.

14. A process as claimed in any of the preceding claims, wherein a carbonyl compound of the formula III where R² and R³ are, independently of one another, hydrogen or alkyl, and R² and R³ are preferably both hydrogen, is reacted.

## Revendications

1. Procédé de préparation d'aminoalcynes à substitution N-alkyle de formule générale I 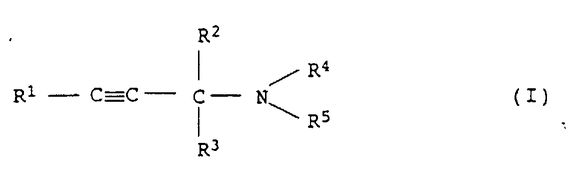 dans laquelle
R¹ représente un atome d'hydrogène, un groupement alkyle halogénoalkyle, cycloalkyle, aryle, alcoxy, alcoxyalkyle ou hydroxyalkyle ;
R² et R³ représentent indépendamment l'un de l'autre un atome d'hydrogène, un groupement alkyle, halogénoalkyle, aryle ou alcoxy ;
R⁴ et R⁵ représentent indépendamment l'un de l'autre un atome d'hydrogène, un groupement alkyle, halogénoalkyle, aryle, alcoxy ou hydroxyalkyle, ou
R⁴ et R⁵ forment ensemble avec l'atome d'azote auquel ils sont liés, un reste hétérocyclique à 5 ou 6 maillons ;
où l'on fait réagir dans une réaction à catalyse hétérogène, un mélange d'un alcyne de formule générale II
R¹― C≡C― H (II)
dans laquelle
R¹ prend la signification susmentionnée,
d'un composé carbonylé de formule générale III 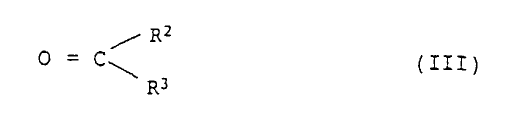 dans laquelle
R² et R³ prennent les significations susmentionnées,
et d'une amine de formule générale IV 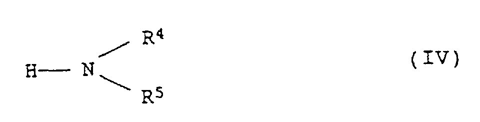 dans laquelle R⁴ et R⁵ prennent les significations susmentionnées, caractérisé en ce que l'on utilise un catalyseur à l'acétylure de cuivre sans support, qui dérive de la malachite et où le catalyseur contient au moins un complexe d'acétylure de cuivre de formule générale V
(CuC₂)_{w} (CH₂O)ₓ (C₂H₂)_{y} (H₂O)_{z} (V)
avec 1 ≤ w, x, y, z < 100,
et un composé du bismuth.

2. Procédé selon la revendication 1, caractérisé en ce que le catalyseur contient 40 à 70% en poids de cuivre et 0,1 à 10% en poids de bismuth.

3. Procédé selon l'une des revendications précédentes, caractérisé en ce que l'on utilise un complexe de formule V dans laquelle
w est mis pour une valeur de 2 à 6,
x est mis pour une valeur de 0,24 à 4,00,
y est mis pour une valeur de 0,24 à 2,40, et
z est mis pour une valeur de 0,67 à 2,8.

4. Procédé selon l'une quelconque des revendications précédentes, caractérisé en ce que l'on mène la réaction sous une pression allant jusqu'à 3 bar, de préférence jusqu'à 2 bar, de façon particulièrement préférée sous la pression environnante.

5. Procédé selon l'une quelconque des revendications précédentes, caractérisé en ce que l'on mène la réaction à une température de 20 à 200°C, de préférence de 30 à 180°C, de façon particulièrement préférée de 40 à 160°C.

6. Procédé selon l'une quelconque des revendications précédentes, caractérisé en ce que la réaction a lieu dans le domaine des pH neutre ou alcalin.

7. Procédé selon l'une quelconque des revendications précédentes, caractérisé en ce que la réaction a lieu dans la masse.

8. Procédé selon l'une quelconque des revendications précédentes, caractérisé en ce que la réaction a lieu dans un milieu non aqueux.

9. Procédé selon l'une quelconque des revendications précédentes, caractérisé en ce que l'on introduit tout d'abord le composé carbonylé de formule III, l'amine de formule IV et le catalyseur, éventuellement dans un solvant, puis on ajoute l'alcyne de formule II, de préférence sous le niveau de liquide du mélange réactionnel.

10. Procédé selon l'une quelconque des revendications précédentes, caractérisé en ce que l'on fait réagir un alcyne de formule II, où R¹ est mis pour un atome d'hydrogène, un groupement alkyle ou hydroxyalkyle.

11. Procédé selon la revendication 10, caractérisé en ce que l'on utilise comme composé de formule II, un alcyne sous forme gazeuse à la température réactionnelle.

12. Procédé selon la revendication 11, caractérisé en ce que l'on utilise comme composé de formule II, de l'acétylène qui n'a été ni comprimé ni liquéfié pour la réaction.

13. Procédé selon l'une quelconque des revendications 1 à 9, caractérisé en ce que l'on fait réagir un alcyne de formule II et une amine de formule VI, où au moins un des restes R¹, R⁴ ou R⁵ est mis pour un groupement hydroxyalkyle.

14. Procédé selon l'une quelconque des revendications précédentes, caractérisé en ce que l'on fait réagir un composé carbonylé de formule III, où R² et R³ sont mis indépendamment l'un de l'autre pour un atome d'hydrogène ou un groupement alkyle, R² et R³ représentant de préférence un atome d'hydrogène tous les deux.
